# FASCICULE DE BREVET EUROPEEN

(11) **EP 2 938 404 B1**
(45) Date de publication et mention de la délivrance du brevet: **28.02.2018**
(21) Numéro de dépôt: 13820831.9
(22) Date de dépôt: 19.12.2013
(51) Int. Cl.: A61Q 19/00, A61K 38/48, A61P 17/00, A61K 8/44

(54) **COMPOSITION COSMETIQUE/ DERMATOLOGIQUE**
KOSMETISCHE / DERMATOLOGISCHE ZUSAMMENSETZUNG
COSMETIC/DERMATOLOGICAL COMPOSITION

(30) Priorité: 31.12.2012 FR 1262977
(43) Date de publication de la demande: 04.11.2015
(73) Titulaire: ACM, 92110 Clichy (FR)
(72) Inventeur: GAUTHIER, Yvon, F-33110 Le Bouscat (FR); BENZEKRI, Laila, Rabat 10100 (MA)
(74) Mandataire: Novagraaf Technologies
(86) Numéro de dépôt international: PCT/FR2013/053196
(87) Numéro de publication internationale: WO 2014/102488

(56) Documents cités:
- OLSSON ET AL: "Leucoderma treated by transplantation of a basal cell layer enriched suspension", BRITISH JOURNAL OF DERMATOLOGY, vol. 138, no. 4, 4 avril 1998 (1998-04-04) , pages 644-648, XP055072218, ISSN: 0007-0963, DOI: 10.1046/j.1365-2133.1998.02177.x
- SANJEEV V. MULEKAR: "Long-term follow-up study of 142 patients with vitiligo vulgaris treated by autologous, non-cultured melanocyte-keratinocyte cell transplantation", INTERNATIONAL JOURNAL OF DERMATOLOGY, vol. 44, no. 10, 1 octobre 2005 (2005-10-01), pages 841-845, XP055072214, ISSN: 0011-9059, DOI: 10.1111/j.1365-4632.2005.02226.x
- SANJEEV V. MULEKAR: "Melanocyte-keratinocyte cell transplantation for stable vitiligo", INTERNATIONAL JOURNAL OF DERMATOLOGY, vol. 42, no. 2, 1 février 2003 (2003-02-01), pages 132-136, XP055072215, ISSN: 0011-9059, DOI: 10.1046/j.1365-4362.2003.01628.x
- MULEKAR SANJEEV V: "Long-term follow-up study of segmental and focal vitiligo treated by autologous, noncultured melanocyte-keratinocyte cell transplantation.", ARCHIVES OF DERMATOLOGY OCT 2004, vol. 140, no. 10, octobre 2004 (2004-10), pages 1211-1215, XP002705994, ISSN: 0003-987X
- GNDP: "Rejuvenating Microdermabrasion", , 1 mars 2012 (2012-03-01), XP002705995, Extrait de l'Internet: URL:http://www.gnpd.com/sinatra/gnpd/searc h_results&search_id=diXKAjDtEh/&p_page_num ber=0&p_page_size=30&item_id=1743748 [extrait le 2013-07-22]
- GNDP: "Freesia & Whipped Vanilla Ultra-Moisturizing Hand and Body Cream", , 1 février 2012 (2012-02-01), XP002705996, Extrait de l'Internet: URL:http://www.gnpd.com/sinatra/gnpd/searc h_results&search_id=diXKAjDtEh/&p_page_num ber=0&p_page_size=30&item_id=1733121 [extrait le 2013-07-22]
- GNDP: "Ultra-Moisturizing Hand and Body Cream", , 1 octobre 2012 (2012-10-01), XP002705997, Extrait de l'Internet: URL:http://www.gnpd.com/sinatra/gnpd/searc h_results&search_id=diXKAjDtEh/&p_page_num ber=0&p_page_size=30&item_id=1906697 [extrait le 2013-07-22]

## Description

### Domaine technique

La présente se rapporte à une composition cosmétique ou dermatologique comprenant un composé chélateur et un composé d'origine végétale possédant une activité protéasique.

La présente invention se rapporte à un procédé de traitement cosmétique comprenant l'utilisation d'une composition cosmétique comprenant un composé chélateur et un composé d'origine végétale possédant une activité protéasique.

La présente invention se rapporte également à l'utilisation d'une composition comprenant un composé chélateur et un composé d'origine végétale possédant une activité protéasique pour le traitement esthétique/cosmétique de la dépigmentation de la peau.

La présente invention se rapporte également à l'utilisation d'une composition comprenant un composé chélateur et un composé d'origine végétale possédant une activité protéasique pour le traitement esthétique du vitiligo.

La présente invention trouve une application notamment dans les domaines cosmétiques et dermatologiques.

Dans la description ci-dessous, les références entre crochets ([]) renvoient à la liste des références présentées à la fin du texte.

### Etat de la technique

L'épiderme est une structure complexe dont les composants cellulaires principaux sont les kératinocytes à l'origine de la production de kératine et de kératohyaline, les cellules de Langerhans qui participent à la défense immunitaire et enfin les mélanocytes dont la fonction principale est la production du pigment mélanique.

La dépigmentation de la peau peut intervenir sur n'importe quel type de peau quelle que soit l'origine ethnique de l'individu. Différents processus peuvent être à l'origine d'une dépigmentation de la peau. Cette dépigmentation peut être acquise survenant dans les suites d"une agression mécanique (dermabrasion), d'une agression chimique ou thermique (brulure thermique) ou d'un traitement par laser.

Il peut s'agir également d'une dépigmentation observée dans le cadre d'une pathologie dépigmentante, par exemple d'un vitiligo qui touche 0,5% de la population mondiale. Cette affection est due à une déperdition chronique des mélanocytes de l'épiderme et des follicules pileux entrainant au bout d'un certain temps l'apparition sur la peau de taches dépigmentées. Le mécanisme exact de la disparition des mélanocytes de l'épiderme n'est pas clairement établi. Plusieurs causes ont été évoquées : un processus auto-immun, l'action nocive de stress psychologiques répétés qui provoqueraient notamment une accumulation toxique pour le mélanocyte de neuromédiateurs ou de radicaux libres (stress oxydatif) dans l'environnement du mélanocyte.

La repigmentation d'une zone/tache dépigmentée localisée est obtenue dans les meilleurs des cas en stimulant la prolifération des mélanocytes résiduels situés dans la paroi des follicules pileux. La « recolonisation » de l'épiderme dépigmenté s'effectue à partir des mélanocytes persistant au niveau des poils noirs qui vont migrer vers l'épiderme. Elle est obtenue suite à un traitement topique ou mieux une irradiation durant plusieurs mois de la zone/tache par les UVB sous surveillance dermatologique.

L'apparition au niveau de l'épiderme de collerettes pigmentées péripilaires est la première manifestation observable du processus de repigmentation. Ces confettis bruns vont s'étaler en surface puis entrer en coalescence au fil des mois de manière à recouvrir plus ou moins complètement la tache dépigmentée. Ce long processus de repigmentation ne peut se manifester qu'au niveau de taches dépigmentées ayant conservé intacts les follicules pileux, c'est-à-dire au niveau des zones au niveau desquelles des poils noirs sont encore présents. Lorsque les follicules pileux sont absents, par exemple dans des zones glabres des extrémités des mains et des pieds, ou dépourvus de mélanocytes (poils blancs) la repigmentation avec les compositions/procédés connus dans l'état de la technique n'est pas possible.

Curieusement, les mélanocytes présents dans l'épiderme pigmenté environnant ne participent pas au processus de repigmentation. Une ébauche de repigmentation, lorsqu'elle se manifeste à partir des bords dépasse rarement quelques millimètres après quelques mois. Si bien qu'en raison de la non implication au processus de repigmentation des mélanocytes des bords, on ne dispose pas à l'heure actuelle de possibilités thérapeutiques pour les lésions dépigmentées glabres et pour les lésions dont les poils sont blancs.

En effet, les procédés et compositions cosmétiques/dermatologiques connus dans l'état de la technique ne permettent pas la repigmentation de zones/taches dépigmentées dépourvues de follicules pileux dont les mélanocytes sont fonctionnels.

Il existe donc un réel besoin dans l'état de la technique de trouver un procédé et/ou une composition palliant ces défauts, inconvénients et obstacles de l'art antérieur, en particulier une composition et/ou procédé permettant de repigmenter la peau, des lésions dépigmentées glabres ou des lésions dont les follicules pileux ont été endommagés ou sont décolorés.

### Description de l'invention

La présente a précisément pour but de répondre aux besoins de l'art en fournissant une composition cosmétique ou dermatologique comprenant un composé chélateur et un composé d'origine végétale ou animale possédant une activité protéasique.

La présente invention a pour objet un procédé de traitement cosmétique de la dépigmentation de la peau et/ou pour restaurer la pigmentation de l'épiderme comprenant l'application sur la peau d'une composition cosmétique comprenant un composé chélateur choisi dans le groupe comprenant la dimercaprolpénicillamine, l'acétopénicillamine, l'acide éthylène diamine tétraacétique (EDTA), l'acide diéthylène triamine penta acétique (DTPA), acide tétraacétique éthylène glycol (EGTA), et un composé d'origine végétale possédant une activité protéasique choisi parmi la broméline ou la papaïne ou animale possédant une activité protéasique choisi parmi la trypsine, la pancréatine, dans laquelle le composé chélateur est à une concentration de 0,01 à 5% en poids par rapport au poids total de la composition.

Les travaux réalisés par les inventeurs ont permis de mettre en avant un nouveau moyen qui permet d'obtenir une autre modalité de repigmentation que ceux connus dans l'état de la technique.

Les travaux réalisés par les inventeurs dans le cadre de l'invention ont permis de mettre en évidence, de manière surprenante, que la composition selon l'invention permet de repigmenter des zones/taches dépigmentées.

En particulier, les travaux réalisés par les inventeurs dans le cadre de l'invention ont permis de mettre en évidence, de manière surprenante, que la composition selon l'invention permet de faciliter la repigmentation des zones/taches dépigmentées en mobilisant les mélanocytes des bords des taches vers la zone dépigmentée.

Dans la présente par « zones/taches dépigmentées » on entend, des zones/taches au niveau de tout ou une partie du corps, par exemple au niveau de la peau, du cuir chevelu présentant un défaut de pigmentation. Il peut s'agir par exemple de zones/taches réparties d'une manière localisées ou généralisées, où les mélanocytes ont partiellement ou totalement disparu ce qui entraine une dépigmentation visible à l'oeil nu.

Selon l'invention, un défaut de pigmentation peut être dû, par exemple à une pathologie, une affection dépigmentante, une agression extérieure, par exemple d'ordre physique, par exemple secondaire à un traitement au laser, par exemple suite à une irradiation par les Ultra Violets (UV), ou enfin une agression chimique. Le défaut de pigmentation peut être du, par exemple, à une affection dépigmentante, ou être secondaire à une agression extérieure, mécanique, thermique ou chimique. Il peut s'agir par exemple soit d'un défaut de pigmentation au niveau de la peau dû à des lésions, soit de lésions dépigmentées glabres ou de lésions, du cuir chevelu, soit enfin de lésions dont les follicules pileux ont été endommagés ou sont décolorés. Il peut s'agir également d'un défaut de pigmentation dû à une pathologie génétique, immunitaire, par exemple tel que le vitiligo, et/ou hormonale.

L'importance du déficit mélanocytaire est variable selon le mécanisme en cause mais aussi selon la précocité ou le caractère tardif de la prise en charge de la dépigmentation. Dans le cadre du vitiligo on observe successivement les phases suivantes pour une zone comportant du duvet ou des poils : tout d'abord une disparition des mélanocytes épidermiques avec conservation des mélanocytes des follicules pileux (poils noirs), puis une disparition conjointe des mélanocytes de l'épiderme et des follicules pileux (poils blancs). Les zones glabres ne disposant pas de cette « réserve mélanocytaire de secours » sont d'emblée rebelles, ne peuvent être traitées par les traitements actuels, car il n'existe pas dans l'état de la technique de moyen permettant de mobiliser les mélanocytes des bords des taches/zones dépigmentées (zones marginales).

Dans la présente par « composé chélateur » on entend un composé capable de former des complexes stables avec une molécule, par exemple des métaux lourds, ou un ion, par exemple le Calcium (Ca) et/ou le Magnésium (Mg), par exemple via des liaisons hydrogènes, des liaisons ioniques. Il peut s'agir, par exemple, d'un composé choisi dans le groupe comprenant la dimercaprolpénicillamine, l'acétopénicillamine, l'acide éthylène diamine tétra-acétique (EDTA), l'acide diéthylène triamine penta acétique (DTPA), acide tétra-acétique éthylène glycole (EGTA).

Selon l'invention, le composé chélateur peut être dans la composition à une concentration de 0,01 à 5% en poids par rapport au poids total de la composition, de 0,05 à 4% en poids par rapport au poids total de la composition, de 1 à 3% en poids par rapport au poids total de la composition.

Avantageusement, le composé chélateur permet, au niveau de la peau une rupture des ponts calciques au niveau des cellules épidermiques induisant ainsi un détachement dermo-épidermique et un élargissement des espaces intercellulaires épidermiques.

Dans la présente par « composé d'origine végétale ou animale possédant une activité protéasique» on entend par exemple, une protéine, une enzyme obtenue et/ou isolée à partir d'un végétal ou d'un animal.

Selon l'invention, le composé possédant une activité protéasique est de préférence un composé d'origine végétale.

Dans la présente par « composé d'origine végétale possédant une activité protéasique » on entend par exemple, une protéine, une enzyme obtenue et/ou isolée à partir d'un végétal, par exemple à partir d'une plante, d'une partie d'une plante, ou d'un fruit. Il peut s'agir par exemple d'un composé obtenu à partir d'un ananas et/ou de la papaye. Il peut d'agir par exemple d'une protéine ayant des propriétés enzymatiques protéolytiques, et/ou d'une protéase, et/ou d'une endoprotéase.

Selon l'invention, le composé d'origine végétale possédant une activité protéasique peut être isolé à partir d'un tissu végétal, provenant d'une plante dans son intégralité et/ou d'une partie d'une plante, par exemple de fruits, feuilles, racines.

Il peut s'agir par exemple d'un composé choisi dans le groupe comprenant la broméline et/ou la papaïne.

Il peut s'agir par exemple de la broméline disponible dans le commerce, par exemple la broméline commercialisée par la société labo Nutrixeal, Planticinal, Nature'sPlus, par exemple sous la forme de poudre, sous la référence catalogue Bromelaine, de broméline extraite de la tige de l'ananas, par exemple la broméline commercialisée par la société Laboratoire IDBIO sous la référence commerciale Bromelaine 1200.

Il peut s'agir par exemple de la papaïne disponible dans le commerce, par exemple la papaïne commercialisée par la société Biovea sous la forme de comprimés comme complément nutritionnel, de papaïne isolée à partir du latex de la papaye.

Dans la présente par « composé d'origine animale possédant une activité protéasique » on entend par exemple, une protéine, une enzyme obtenue et/ou isolée à partir d'un mammifère. Il peut s'agir par exemple d'une protéine, d'une enzyme obtenue et/ou isolée à partir d'un échantillon de tissu, d'un liquide biologique, par exemple du suc pancréatique.

Le composé d'origine animale possédant une activité protéasique peut être par exemple une endoprotéase, par exemple la trypsine, la pancréatine.

Les travaux réalisés par les inventeurs ont permis de montrer de manière surprenante que, avantageusement, les protéases d'origines végétales ont une activité protéolytique comparable à celles des protéases animales, par exemple la trypsine, en particulier vis-à-vis de la coagulation du lait.

Selon l'invention, le composé d'origine végétale ou possédant une activité protéasique peut être dans la composition à une concentration de 0,001 à 10% en poids par rapport au poids total de la composition, de 0,005 à 4% en poids par rapport au poids total de la composition, de 0,01 à 1% en poids par rapport au poids total de la composition.

Selon l'invention, le composé d'origine végétale ou possédant une activité protéasique peut être dans la composition à une concentration de 3 à 30000 en activité enzymatique (Unité de digestion de gélatine (GDU)), de 15 à 12000 GDU, de 30 à 3000 GDU.

Selon l'invention, le composé d'origine végétale ou animal possédant une activité protéasique peut être dans la composition à une concentration de 0,001 à 10% en poids par rapport au poids total de la composition, de 0,005 à 4% en poids par rapport au poids total de la composition, de 0,01 à 1% en poids par rapport au poids total de la composition.

Selon l'invention, la composition cosmétique peut être sous toute forme connue de l'homme du métier dans les domaines de la cosmétique et de la dermatologie, sans aucune restriction galénique particulière.

La composition cosmétique ou dermatologique peut être sous toute forme galénique connue de l'homme du métier, par exemple sous la forme d'un onguent, d'un sérum, d'une crème, d'une huile, d'un lait, d'une pommade, d'une poudre, d'un stick, d'un tampon imbibé, d'un patch transdermique, d'une solution, d'un gel, d'un spray, d'une lotion ou d'une suspension. Il peut s'agir par exemple d'une crème, d'un lait, d'un gel, d'une lotion, d'un onguent, d'un patch transdermique, un baume, un masque.

Selon l'invention, quel(s) que soi(en)t l'extrait ou le mélange d'extraits utilisés, la composition cosmétique ou dermatologique de la présente invention peut comprendre en outre au moins un agent actif additionnel choisi, par exemple parmi un agent anti-âge, un agent hydratant, un agent apaisant et/ou un mélange de ces agents. Ces agents peuvent être ceux couramment utilisés dans les produits cosmétiques et/ou dermatologiques. Ce ou ces agent(s) peuvent être utilisé(s) dans la composition de la présente invention par exemple aux concentrations usuellement utilisées et connues de l'homme du métier dans les compositions cosmétiques ou dermatologiques

La composition cosmétique selon l'invention peut comprendre un ou plusieurs adjuvants connus de l'homme du métier. Il peut s'agir par exemple d'un ou plusieurs adjuvant(s) choisi(s) parmi des agents de type esters, des agents hydratants, des agents émollients, des agents épaississants minéraux, des filtres solaires minéraux, des parfums, des conservateurs, des vitamines et des provitamines, des protéines, des agents séquestrants, des agents alcanisants, des agents acidifiants, des agents réducteurs, des agents oxydants, des charges minérales, des colorants naturels ou tout autre adjuvant pouvant être cité dans le dictionnaire INCI (International Nomenclature Cosmetic Ingredients) publié par le CTFA (Cosmetic, Toiletry and Fragrance Association).

Selon l'invention, la composition peut être une composition cosmétique antiâge.

Les inventeurs de la présente invention sont les premiers à avoir démontré de manière surprenante et inattendue que la composition selon l'invention permet une repigmentation de la peau.

En particulier. les inventeurs ont démontré que la composition de la présente décrite permet un élargissement des espaces intercellulaires épidermiques permettant, notamment la migration mélanocytaire.

Les inventeurs sont également les premiers à avoir démontré que l'amélioration de la migration mélanocytaire avec la composition décrite permet avantageusement d'initier le processus de repigmentation de la peau.

En outre, les inventeurs sont les premiers à avoir montré que la composition décrite permet la repigmentation de zone/tache dépigmentée, quel que soit leur origine ou cause.

En outre, les inventeurs de la présente invention ont également montré que la composition décrite permet de repigmenter les follicules pileux, par exemple les poils, les cheveux, la barbe etc.

En outre, les inventeurs dans le cadre de la présente invention ont également mis en évidence, de manière surprenante que la composition décrite permet de repigmenter la peau, notamment les taches/zones dépigmentées dues au vitiligo.

En particulier, les inventeurs dans le cadre de la présente invention ont également mis en évidence, de manière surprenante que la composition décrite permet de faciliter la repigmentation marginale des taches/zones dépigmentées dues au vitiligo.

En particulier, les inventeurs ont démontré que la composition de la présente permet avantageusement un élargissement des espaces intercellulaires épidermiques permettant, notamment la migration centripète des mélanocytes à partir de la zone normalement pigmentée vers la zone dépigmentée. Les mélanocytes peuvent être avantageusement stimulés par application/irradiation avec des ultra-violets (UV), par exemple UVB ou UVA, et/ou exposition aux infrarouges (IR).

Les inventeurs sont les premiers à avoir montré une migration mélanocytaire à partir des bords de zones/taches dépigmentées.

Les inventeurs sont également les premiers à avoir démontré que la facilitation de la migration mélanocytaire à partir des bords avec la composition décrite lorsqu'elle est associée à l'utilisation des UV permet avantageusement d'initier le processus de repigmentation centripète des taches dépigmentées à partir des bords.

En outre, les inventeurs sont les premiers à avoir montré que la composition décrite en association avec l'exposition solaire ou l'exposition aux UVB ou UVA ou aux infrarouges (IR), permet avantageusement d'obtenir ce type de repigmentation marginale de zone/tache dépigmentée, quelle que soit son origine ou sa cause.

En outre, les inventeurs de la présente invention ont également montré que la composition décrite permet aussi d'accroitre la repigmentation épidermique périfolliculaire, par exemple en élargissant les espaces intercellulaires.

Ainsi, la présente invention se rapporte à l'utilisation cosmétique ou dermatologique d'une composition décrite pour repigmenter taches/zones dépigmentées.

En particulier, la repigmentation est réalisée à partir des bords des taches/zones dépigmentées.

Les taches/zones dépigmentées sont telles que définies précédemment.

La présente invention a également pour objet la composition décrite pour le traitement cosmétique de la dépigmentation de la peau et/ou pour restaurer la pigmentation de l'épiderme.

Dans la présente, par « traitement cosmétique de la dépigmentation » on entend une diminution progressive de la surface de la zone dépigmentée pouvant aboutir jusqu'à sa disparition totale.

Dans la présente, par « restauration de la dépigmentation » on entend une disparition progressive puis totale des zones/taches dépigmentées, par exemple siégeant à un endroit donné du tégument/ de la peau.

En d'autres termes, dans la présente, par « restauration de la pigmentation » on entend une disparition progressive puis totale des zones/taches dépigmentées, par exemple siégeant à un endroit donné du tégument/ de la peau.

La présente invention a également pour objet la composition décrite pour stimuler la pigmentation, notamment de la peau, du cuir chevelu et/ou des follicules pileux

La présente invention a également pour objet l'utilisation cosmétique de la composition décrite pour stimuler la pigmentation de la peau, du cuir chevelu et/ou des follicules pileux, notamment au niveau d'une tache/ zone de dépigmentée.

En particulier, la présente invention a également pour objet l'utilisation cosmétique de la composition décrite pour faciliter la repigmentation marginale à partir des bords des taches dépigmentées siégeant au niveau de la peau et du cuir chevelu.

La présente invention a également pour objet l'utilisation cosmétique de la composition décrite pour repigmenter la peau du cuir chevelu et/ou des follicules pileux, notamment au niveau de taches/ zones dépigmentées.

La présente invention a également pour objet un procédé cosmétique comprenant l'application sur la peau d'une composition cosmétique selon l'invention, par exemple il peut s'agir d'un soin cosmétique ou d'un traitement cosmétique pour stimuler la pigmentation, par exemple de la peau, du cuir chevelu et/ou des follicules pileux, notamment au niveau d'une tache/zone de dépigmentée.

Dans la présente par « soin cosmétique » on entend par exemple l'application cutanée d'une composition selon l'invention.

La présente invention a également pour objet un procédé de traitement cosmétique comprenant l'application sur la peau d'une composition cosmétique telle que définie ci-dessus.

Selon l'invention, le traitement cosmétique peut-être un traitement pour stimuler la pigmentation de la peau, pour unifier la pigmentation de la peau. Il peut s'agir également d'un procédé pour stimuler la pigmentation des follicules pileux, par exemples des cheveux, des poils, de la barbe.

Dans la présente, le procédé cosmétique/le traitement cosmétique sont des procédés/traitements non thérapeutiques.

Pour cette application, on peut, par exemple, utiliser les formes galéniques décrites ci-dessus. L'application sur la peau peut donc être réalisée en fonction de la forme galénique utilisée.

L'application peut être réalisée avec une quantité suffisante de la composition afin que toute la surface de la peau à traiter soit traitée. Il peut s'agir par exemple d'une application classique, comme une crème sur la peau.

En particulier, le procédé de traitement peut comprendre l'application de la composition décrite sur la zone marginale, par exemple à 1cm de part et d'autre de la limite séparant la zone pigmentée et dépigmentée, avec une quantité suffisante de la composition.

L'application peut être également accompagnée d'une exposition aux rayonnements, par exemple ultra-violet (UV), par exemple aux UVB ou UVA ou aux infrarouges (IR). L'exposition peut être réalisée, par exemple lors de l'application de la composition et/ou à différent temps après l'application, par exemple après une application quotidienne, biquotidienne, hebdomadaire et/ou bi-mensuelle.

Une trousse de soin cosmétique comprenant une composition cosmétique selon la présente invention et un manuel ou une notice d'utilisation. La trousse peut être par exemple un conditionnement est également décrite.

Le manuel ou cette notice peut avoir notamment pour fonction d'expliquer à l'utilisateur la manière et la fréquence d'application sur la peau la composition de la présente invention.

La trousse de soin cosmétique peut également comprendre un applicateur, par exemple une spatule, une brosse, un applicateur dynamique, par exemple un dispositif de massage imprégné de la composition cosmétique décrite.

D'autres avantages pourront encore apparaître à l'homme du métier à la lecture des exemples ci-dessous, illustrés par les figures annexées, donnés à titre illustratif.

### Brève description des figures

- La figure 1 représente une photographie d'une image au microscope du clivage entre l'épiderme et le derme après incubation dans une solution contenant des chélateurs chimiques (Solution d'EDTA).
- La figure 2 représente une photographie d'une image prise au microscope de la déstructuration de l'épiderme après incubation en présence de protéases d'origine animale (Trypsine 0,25%).
- La figure 3 représente une photographie d'une image réalisée au microscope de la déstructuration de l'épiderme après incubation en présence de protéases d'origine végétale (Broméline 0,5%).
- La figure 4 représente une photographie réalisée au microscope :
   ∘ A (1/2) Migration mélanocytaire dans espace élargi entre l'épiderme et le derme.
   ∘ B (2/2) Espace de migration ménagé entre épiderme et derme après application topique d'une composition contenant 3% d'EDTA.
- La figure 5 représente une photographie au microscope de l'épiderme avec des espaces intercellulaires élargis après incubation en présence de broméline.
- La figure 6 représente une photographie d'un poignet avec une peau comprenant une zone dépigmentée. La flèche indique la zone de repigmentation suite à l'application de la composition selon l'invention.

### EXEMPLES

### Exemple 1 : Composition comprenant des protéases d'origine végétale et au moins un composé chélateur

La composition comprenait les éléments suivants :
3 grammes d'EDTA, avec 1 g de Broméline poudre ont été incorporés QS/ 100g de Néribase crème (marque déposée) commercialisée par la société Bayer Santé.

La composition de la Néribase crème utilisée est la suivante : Stéarate de polyoxyéthylèneglycole, alcool stéarylique, paraffine liquide, vaseline, édétate de sodium, polymère carboxyvinylique, hydroxyde de sodium, parahydroxybenzoate de méthyle 0,07 %, parahydroxybenzoate de propyle 0,03 %, eau purifiée.

### Exemple 2 : application de la composition comprenant des protéases d'origine végétale et au moins un composé chélateur in vitro et in vivo

### Matériel et méthode

### 1. Etude préliminaire in vitro de la dissociation de l'épiderme en immersion avec différentes substances

Les fragments cutanés de 4mm testés ont été fournis par un service de chirurgie plastique. Après dégraissage avec des ciseaux fins, ils ont été mis à incuber pendant 18 h à 4°C dans des boites de Pétri contenant respectivement quelques ccs des solutions suivantes :
- soit Trypsine 0,25% (commercialisé par la société Eurobio),
- soit Pancréatine (commercialisé par la société Sigma Chimie) solution à 1% dans un tampon phosphate salin à (PBS) dont la composition est la suivante: 137mM NaCl, 2,7mM Kl, 10mM Na₂HPO₄, 1,76mM KH₂PO₄.
- soit une solution comprenant 3% d'EDTA ou d'EGTA (commercialisée par la société Sigma Chimie) dans un tampon phosphate PBS,
- soit une solution de Broméline à 1% dans un tampon phosphate PBS,
- Soit une solution comprenant 3% d'EDTA et 1% Broméline

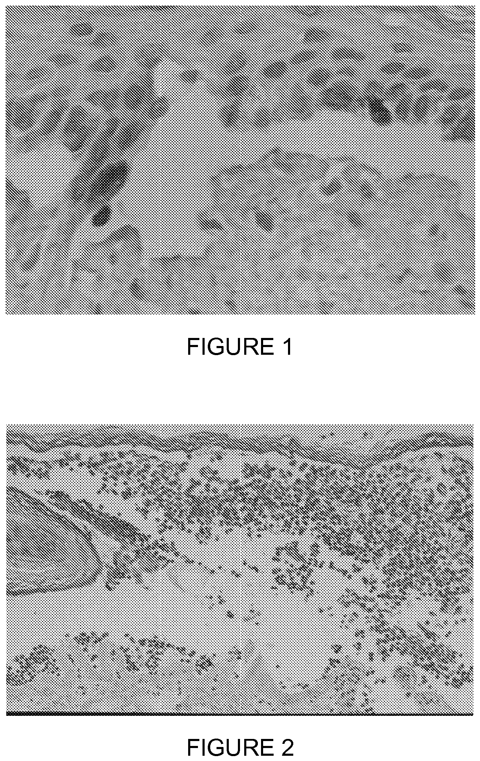

L'EDTA, ainsi que l'EGTA sont des substances dont l'activité chélatrice est connue. Elles favorisent la dissociation *in vitro* de l'épiderme par leur action chélatrice sur le Ca et le Mg entrainant une rupture des ponts calciques. La figure 1 représente une photographie au microscope du clivage entre l'épiderme et le derme après incubation dans une solution contenant des chélateurs chimiques (Solution d'EDTA) coloration Hématéine-Eosine-Safran (HES) le marqueur de l'HMB45 en utilisant un anticorps monoclonal commercialisé par la société ABCAM sous la référence commerciale Antimelanoma Antibody.

Le procédé d'incubation et de coloration mis en oeuvre est celui décrit dans HISTOLOGIE DE LA PEAU NORMALE : B.Cribier, E. Grosshans Encyclopédie médicochirurgicale 98-0856A-10 ED ELSEVIER [5].

Comme montré sur la figure 1, il a été observé un clivage anatomique au niveau de la membrane basale permettant une séparation facile à la pince du derme de l'épiderme.

### 1.1. Les protéases

### Les Protéases d'origine animale

Après une immersion durant 18 heures à 4°C dans une solution de trypsine à 0,25% (commercialisé par la société Eurobio) ou une solution de Pancréatine) à 1%, il a été observé une destructuration complète de l'épiderme avec une protéolyse des ponts d'union existant entre les cellules épidermiques et les mélanocytes. De plus il a été montré un clivage intraépidermique suprabasal tel que représenté sur la figure 2.

La figure 2 représente une photographie au microscope de la déstructuration de l'épiderme après incubation en présence de protéases d'origine animale (Trypsine 0,25%) avec une coloration HES.

Cette exemple démontre bien que la trypsine a *in vitro* une activité protéolytique qui aboutit à la rupture des zones de jonction intercellulaire de l'épiderme, libérant les kératinocytes et les mélanocytes et entrainant par ailleurs un clivage intra épidermique suprabasal.

### Les protéases d'origine végétale

Les fragments cutanés ont été immergés dans une solution de Broméline ou de Papaine à 1% durant 18h à 4°C. Après cette incubation, la désorganisation de l'épiderme était similaire à celle précédemment observée avec la trypsine. Il existe une déstructuration de l'épiderme avec un clivage suprabasal.

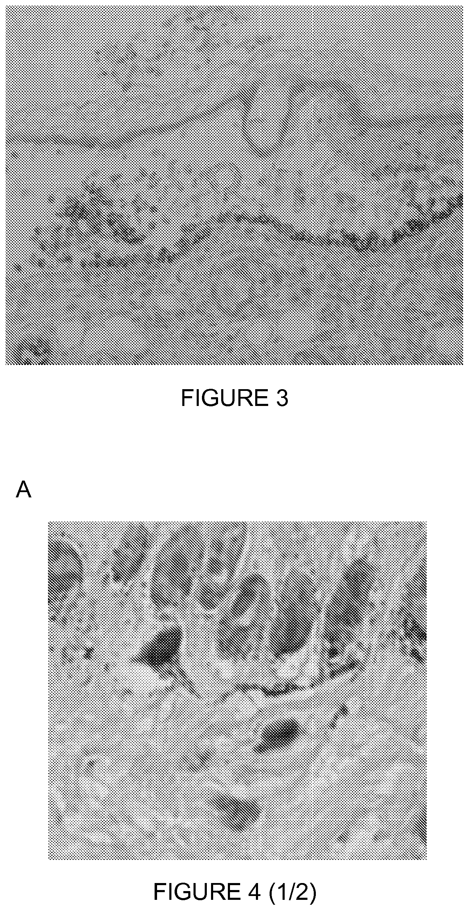

La figure 3 représente une photographie d'une image au microscope de la déstructuration de l'épiderme après incubation en présence de protéases d'origine végétale (Broméline à 0,5%) avec une coloration avec HES.

Tel que démontré dans cette exemple, la broméline ainsi que la papaïne ont une activité comparable à celle des protéases animales au niveau des ponts d'union des cellules épidermiques qui sont digérés et rompus.

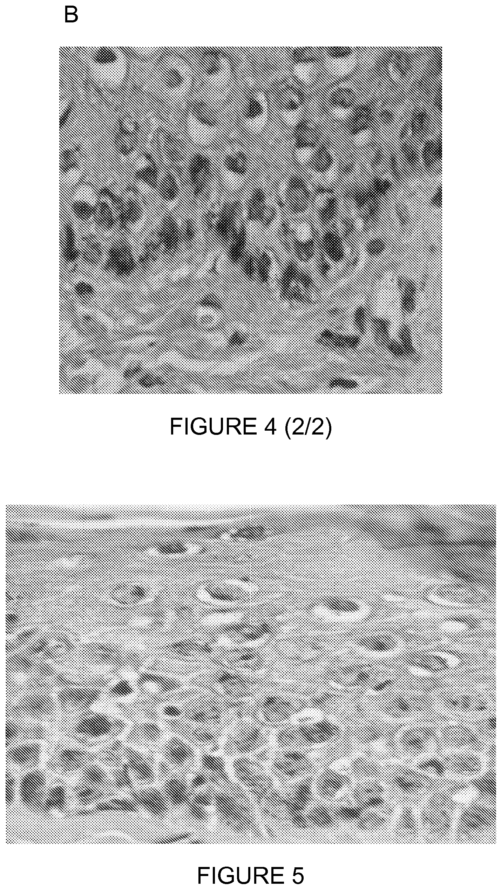

*In vivo* l'application topique sur la peau d'une composition contenant 1% de Broméline a provoqué un élargissement des espaces inter cellulaires tel que représenté sur la figure 5. La figure 5 représente une photographie au microscope de l'épiderme avec des espaces intercellulaires élargis après incubation en présence de broméline.

Les procédés d'incubation et de coloration des cellules étaient similaires à ceux utilisés pour les protéases animales. HISTOLOGIE DE LA PEAU NORMALE : B.Cribier, E. Grosshans Encyclopédie médicochirurgicale 98-0856A-10 ED ELSEVIER [5].

Tel que représenté sur ces figures, les protéases d'origine animale à savoir la pancréatine et la trypsine, ou d'origines végétales à savoir la broméline extraite de l'ananas, ou la papaïne extraite de la papaye, ont un mécanisme d'action commun sur les jonctions intercellulaires et des effets semblables au niveau de l'épiderme. En particulier, ces protéases permettent un clivage en général suprabasal, et une fragilité des liaisons intercellulaires portant sur l'intégralité des cellules de l'épiderme. Une simple agitation mécanique suffit pour libérer toutes ces cellules et obtenir ainsi une suspension cellulaire utilisable pour la réalisation de cultures.

### 2. Etude expérimentale chez le cobaye de l'action des substances chimiques chélatrices (EDTA) par voie topique sur la migration mélanocytaire

Dans cet exemple, le cobaye dans sa variété « piebald » présente d'une manière contiguë des zones cutanées noires possédant des mélanocytes et des zones blanches dépourvues de mélanocytes.

Le cobaye piebald est un modèle animal expérimental reconnu pour étudier la repigmentation et en particulier des avancées pigmentaires en zone dépourvue de mélanocytes.

Dans cet exemple, des tests successifs ont été réalisés sur la zone jonctionelle séparant la zone pigmentée de la zone dépigmentée.

En particulier, des préparations topiques contenant indépendamment des substances chimiques chélatrices, et/ou des protéases à savoir : préparation topique contenant 3% EDTA dans Néribase comme excipient ont été appliquées sur la zone jonctionelle, à cheval sur zone pigmentée et dépigmentée. La composition a été appliquée quotidiennement au même endroit pendant un mois. Parallèlement une irradiation par les UVB de cette zone a été réalisée tous les jours selon la progression de la durée d'irradiation suivante : 30 secondes le premier et le deuxième jour avec augmentation tous les deux jours de 15 secondes jusqu'à atteindre 4 minutes avec l'appareil suivant : Skinlight SL UVB 311 DAVITA Germany.

Pour objectiver une éventuelle avancée pigmentaire des photos avec calques, ainsi qu'un tatouage parallèle aux limites de la zone pigmentée, à une distance de 5 mm, ont été réalisés.

Suite à l'application de la composition comprenant 3% d'EDTA, une avancée pigmentaire objectivable a été observée au bout d'un mois. Toutefois, aucun effet n'a été observé avec la composition comprenant de l'EGTA seul.

En outre un examen histologique a été effectué après fixation, inclusion dans la paraffine et coloration (HES) Le procédé mis en oeuvre est celui décrit dans HISTOLOGIE DE LA PEAU NORMALE : B.Cribier, E. Grosshans Encyclopédie médicochirurgicale 98-0856A-10 ED ELSEVIER [5]. La figure 4 représente une photographie de la coupe histologique de l'épiderme obtenue après application de l'EDTA.

Comme montré sur la figure 4, l'applications d'une composition comprenant de l'EDTA a permis d'élargir l'espace situé entre la membrane basale et la couche basale de l'épiderme et a crée de ce fait un espace de migration pour les mélanocytes.

### 3. Effet in vivo d'une composition comprenant un composé chélateur et un composé d'origine végétale possédant une activité protéasique.

L'expérience réalisée au point 2 précitée a été répétée avec une composition comprenant EDTA 3% et de la Broméline à 1% dans de l'excipient Néribase.

Lors de cette expérience la composition comprenant de l'EDTA et de la Broméline a été appliquée selon le procédé décrit au point 2 ci-dessus de façon topique.

L'application topique de cette composition a favorisé de manière significative et synergique l'avancée pigmentaire et donc la repigmentation chez le cobaye (résultats non fournie).

En outre, il a été démontré que la composition comprenant à la fois de l'EDTA et de la broméline permet d'avoir un effet synergique sur la repigmentation de la peau.

### Exemple 3 : effet in vivo d'une composition comprenant une protéase d'origine végétale et au moins un composé chélateur.

Dans cet exemple, des compositions comprenant de 1 à 3% d'EDTA et 1% de broméline ont été appliquées après consentement éclairé sur la peau de patients volontaires ayant un vitiligo.

Les compositions ont été réalisées avec une base cosmétique, la Neribase tel que décrit dans l'exemple 1 : Stéarate de polyoxyéthylèneglycole, alcool stéarylique, paraffine liquide, vaseline, édétate de sodium, polymère carboxyvinylique, hydroxyde de sodium, parahydroxybenzoate de méthyle 0,07 %, parahydroxybenzoate de propyle 0,03 %, eau purifiée.

Chaque composition a été appliquée une fois par jour pendant 2 mois au niveau des bords pigmentés entourant les taches dépigmentées glabres, sur les pieds ou les poignets. La composition a été également appliquée au niveau des bords pigmentés entourant des poils blancs dépigmentées.

En outre, des patients ont suivi une photothérapie par UVB : 2 fois par semaine avec application de la composition 2 fois par jour pendant 2 mois ou une exposition solaire, 3 fois par semaine, avec application de la composition 2 fois par semaine pendant 2 mois

Des photos ont été prises à l'aide d'un appareil photo numérique E-620 OLYMPUS régulièrement tous les 15 jours pour suivre l'évolution de la repigmentation à partir des bords sur les zones sur lesquelles la composition a été appliquée

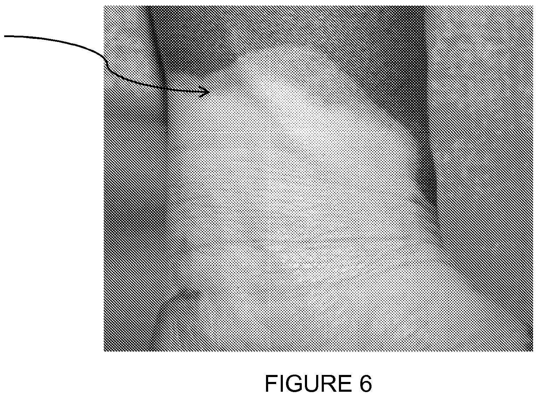

La figure 6 est une photographie prise au bout d'un mois de traitement avec la composition comprenant de l'EDTA 3%, de la Broméline 1% dans du Néribase. Telle que montrée sur cette figure, une repigmentation conséquente avec une avancée de plus un centimètre (indiquée par une flèche sur la figure) à partir des bords des taches dépigmentées a été observée.

Aucune repigmentation n'a été observée pour les patients pour lesquels seul un traitement photothérapie a été appliqué.

Tel que démontré dans cet exemple, la composition comprenant une substance chélatrice associée à une protéase, en particulier une protéase d'origine végétale permet la migration mélanocytaire et permet avantageusement de favoriser la repigmentation de taches dépigmentées de la peau.

La composition utilisée permet donc un traitement de la dépigmentation de la peau.

En outre, tel que démontré dans cet exemple, la composition utilisée associée aux UV permet en outre d'améliorer le traitement des taches de dépigmentation dues au vitiligo et d'augmenter significativement la repigmentation.

### Listes des références

1 : Cahn RD, Coon HG,Cahn MB. (1967). Cell culture and cloning techniques in : Methods in Developmental Biology, eds. Crowell New-York, pp 493-530.
2 : Takeishi M, Okada TS (1972). Role of magnesium and calcium ions in cell-to-substrate adhesion. Exp.Cell Res 74 : 51-60.
3 :Vielkind U, Crawford BJ.Evaluation of different procedures for the dissociation of retinal pigmented epithelium into single viables cells. Pigment Cell Research (1988) 1 : 419-433.
4 : Baker KW, Habowsky EJ. EDTA separation and ATPase Langerhans Cell Staining in the mouse Epidermis. J Invest of Dermatol. 1983 ;80 (2) :104-107
5 : HISTOLOGIE DE LA PEAU NORMALE: B.Cribier, E. Grosshans Encyclopédie médicochirurgicale 98-0856A-10 ED ELSEVIER

## Revendications

1. Procédé de traitement cosmétique de la dépigmentation de la peau et/ou pour restaurer la pigmentation de l'épiderme comprenant l'application sur la peau d'une composition cosmétique comprenant un composé chélateur choisi dans le groupe comprenant la dimercaprolpénicillamine, l'acétopénicillamine, l'acide éthylène diamine tétraacétique (EDTA), l'acide diéthylène triamine penta acétique (DTPA), acide tétraacétique éthylène glycol (EGTA), et un composé d'origine végétale possédant une activité protéasique choisi parmi la broméline ou la papaïne ou animale possédant une activité protéasique choisi parmi la trypsine, la pancréatine, dans laquelle le composé chélateur est à une concentration de 0,01 à 5% en poids par rapport au poids total de la composition.

2. Procédé selon la revendication 1 dans lequel le composé chélateur est l'acide éthylène diamine tétraacétique (EDTA).

3. Procédé selon l'une quelconque des revendications 1 à 2, dans lequel le composé chélateur est à une concentration de 1 à 3% en poids par rapport au poids total de la composition.

4. Procédé selon l'une quelconque des revendications 1 à 3, dans lequel le composé d'origine végétale possédant une activité protéasique est à une concentration de 0,01 à 10% en poids par rapport au poids total de la composition.

5. Procédé selon l'une quelconque des revendications 1 à 4, dans lequel le composé d'origine végétale possédant une activité protéasique est à une concentration de 0,01 à 1% en poids par rapport au poids total de la composition.

6. Procédé selon l'une quelconque des revendications 1 à 5, dans lequel ladite composition est sous l'une des formes choisies parmi une crème, un lait, une pommade, une lotion, un gel, une huile, un baume, un onguent un gel, un masque.

7. Procédé selon l'une quelconque des revendications 1 à 6, dans lequel ladite composition est pour son utilisation dans le traitement cosmétique pour restaurer la pigmentation de la peau.

8. Procédé selon l'une quelconque des revendications 1 à 6, dans lequel ladite composition est pour son utilisation dans le traitement cosmétique pour restaurer la pigmentation de la peau au niveau d'une tache de dépigmentation.

## Patentansprüche

1. Verfahren zur kosmetischen Behandlung der Depigmentierung der Haut und/oder zum Wiederherstellen der Pigmentierung der Epidermis umfassend das Auftragen auf der Haut einer kosmetischen Zusammensetzung, die eine chelatbildende Verbindung ausgewählt aus der Gruppe die Dimercaprolpenicillamin, Acetopenicillamin, Ethylendiamintetraessigsäure (EDTA), Diethylentriaminpentaessigsäure (DTPA), Ethylenglykoltetraessigsäure (EGTA), und eine Verbindung pflanzlichen Ursprungs mit einer Protease-Aktivität, ausgewählt aus Bromelain oder Papain, oder tierischen Ursprungs mit einer Protease-Aktivität, ausgewählt aus Trypsin und Pankreatin, umfasst, worin die chelatbildende Verbindung in einer Konzentration von 0,01 bis 5 Gewichts-% bezogen auf das Gesamtgewicht der Zusammensetzung vorliegt.

2. Verfahren nach Anspruch 1, wobei die chelatbildende Verbindung Ethylendiamintetraessigsäure (EDTA) ist.

3. Verfahren nach einem der Ansprüche 1 bis 2, wobei die chelatbildende Verbindung in einer Konzentration von 1 bis 3 Gewichts-% bezogen auf das Gesamtgewicht der Zusammensetzung vorliegt.

4. Verfahren nach einem der Ansprüche 1 bis 3, wobei die Verbindung pflanzlichen Ursprungs mit einer Protease-Aktivität in einer Konzentration von 0,01 bis 10 Gewichts-% bezogen auf das Gesamtgewicht der Zusammensetzung vorliegt.

5. Verfahren nach einem der Ansprüche 1 bis 4, wobei die Verbindung pflanzlichen Ursprungs mit einer Protease-Aktivität in einer Konzentration von 0,01 bis 1 Gewichts-% bezogen auf das Gesamtgewicht der Zusammensetzung vorliegt.

6. Verfahren nach einem der Ansprüche 1 bis 5, wobei die Zusammensetzung in einer der Formen vorliegt, die ausgewählt ist aus einer Creme, einer Milch, einer Salbe, einer Lotion, einem Gel, einem Öl, einem Balsam, einer Paste, einem Gel, einer Maske.

7. Verfahren nach einem der Ansprüche 1 bis 6, wobei die Zusammensetzung für ihre Verwendung zur kosmetischen Behandlung der Wiederherstellung der Pigmentierung der Haut ist.

8. Verfahren nach einem der Ansprüche 1 bis 6, wobei die Zusammensetzung für ihre Verwendung zur kosmetischen Behandlung der Wiederherstellung der Pigmentierung der Haut im Bereich eines depigmentierten Flecks ist.

## Claims

1. Method of cosmetic treatment of the depigmentation of the skin and/or for restoring the epidermis pigmentation comprising applying onto the skin a cosmetic composition comprising a chelating compound selected in the group including the dimercaprolpenicillamine, the acetopenicillamine, ethylene tetraacetic diamine acid (EDTA), diethylene triamine penta acetic acid (DTPA), tetraacetic ethylene glycol acid (EGTA), and a compound of vegetable origin having an activity proteasic selected among the bromeline or papain or animal having a proteasic activity selected among trypsin, the pancreatin, in which the chelating compound is at a concentration from 0.01 to 5% in weight compared to the total weight of the composition.

2. Method according to the claim 1 wherein the chelating compound is the ethylene tetraacetic diamine acid (EDTA).

3. Method according to any of the claims 1 to 2, wherein the chelating compound is with a concentration from 1 to 3% in weight compared to the total weight of the composition.

4. Method according to any of the claims 1 to 3, wherein the compound of vegetable origin having a proteasic activity is with a concentration from 0.01 to 10% in weight compared to the total weight of the composition.

5. Method according to any of the claims 1 to 4, wherein the compound of vegetable origin having a proteasic activity is with at a concentration from 0.01 to 1% in weight compared to the total weight of the composition.

6. Method according to any of claims 1 to 5, wherein said composition is under one of the forms selected among a cream, a milk, a pomade, a lotion, a gel, an oil, a balsam, an ointment a gel, a mask.

7. Composition according to any of claims 1 to 6, wherein said composition is for use in the cosmetic treatment for restoring the pigmentation of the skin.

8. Composition according to any of claims 1 to 6, wherein said composition is for use in the cosmetic treatment for restoring the pigmentation of the skin on the level of a spot of depigmentation.
